# EUROPEAN PATENT APPLICATION

(11) **EP 4 328 291 A1**
(43) Date of publication of application: **28.02.2024**
(21) Application number: 21942372.0
(22) Date of filing: 28.05.2021
(51) Int. Cl.: C12M 1/00, C12M 1/38

(54) **POLYMERASE CHAIN REACTION DEVICE**

(71) Applicant: Wang, Chin Hung, Taipei, Taiwan 105 (TW)
(72) Inventor: Wang, Chin Hung, Taipei, Taiwan 105 (TW)
(74) Representative: Engelhard, Markus
(86) International application number: PCT/CN2021/096731
(87) International publication number: WO 2022/246797

(57) **Abstract**

Provided is a PCR device including a conveying module and a heat supply module. The conveying module includes: a bench; a rail being loop-style, allowing the bench to move along the rail; and at least one holder arranged on the bench, allowing at least one reactor tube to be detachably disposed at the at least one holder. The heat supply module includes at least one heat supply block for adjusting the temperature of the at least one reactor tube.

## Description

### BACKGROUND OF THE INVENTION

### FIELD OF THE INVENTION

The present disclosure relates to an experiment-oriented reaction device and a reaction method, and more particularly to a polymerase chain reaction (PCR) device that shortens the before-reaction loading waiting time for samples, has a common total heat supply duration, and has a common heat-transfer medium.

### DESCRIPTION OF THE PRIOR ART

Polymerase chain reaction (PCR) technology involves repeatedly heating and cooling a sample solution that contains nucleic acids and thereby exponentially expanding the nucleic acids with specific base sequence regions. The technique of expanding nucleic acids is usually applied to life science, genetic engineering and medicine to conduct analyses and make diagnoses.

A variety of PCR devices are commercially available to meet user needs. In basic laboratories, collected samples are usually added to micro test tubes, and then the micro test tubes are placed in a PCR device in which the samples simultaneously, jointly undergo a cycle of expanding the nucleic acids. Therefore, these types of batch reaction devices are suitable for academic research that not only requires a large number of samples but also entails comparing and analyzing multiple samples.

However, medical testing carried out for the purpose of clinical therapy is often confronted with an emergency that necessitates receiving a testing result immediately to decide on a subsequent, necessary therapeutic procedure (STAT for short). If a reaction is carried out by batch with a PCR device, with a sample lying on a bench and undergoing the reaction, an incoming STAT sample to be tested must wait its turn to be loaded to undergo the reaction, causing the STAT sample to spend much time receiving a testing result. Specifically speaking, the total time taken to receive a testing result includes the time the STAT sample takes to undergo the reaction and the time the STAT sample takes to wait its turn. In a severe scenario, a testing result is not received in time, delaying the therapeutic procedure to be given to the patient-owner of the sample. Moreover, STAT samples are often urgent cases and rarely come in at the same time. Therefore, only a small number of samples are allowed to be loaded to undergo the reaction carried out by batch with the PCR device with a view to receiving a testing result as soon as possible, albeit at the cost of reduced equipment efficiency and increased power consumption.

As mentioned above, not only is it urgent to receive the testing results about STAT samples, but the STAT samples also come in at different timepoints, precluding the collective loading of samples upon completion of the collection of all the samples. To address these issues, it is provided a conventional PCR device that has a plurality of sample chambers for holding samples and allowing the samples to separately undergo a reaction. Specifically speaking, not only are the samples added to the plurality of sample chambers at different timepoints, but it is also feasible to set different heat supply temperatures, durations or numbers of cycles according to the timepoints at which the samples are added to the sample chambers and the types of reactions the samples are to undergo. However, the aforesaid large-sized PCR device disclosed according to prior art is equivalent to a combination of a plurality of small-sized PCR devices. Since the plurality of small-sized PCR devices carry out a reaction separately, have different heat supply blocks, and have different heat supply modes, the plurality of small-sized PCR devices lack a common heat supply module, resulting in an overly high power of the large-sized PCR device. Moreover, with the plurality of small-sized PCR devices undergoing a reaction separately, it is difficult to perform quality control over them.

Therefore, it is imperative to provide a PCR device conducive to the earlier loading of samples to a bench to undergo a reaction, the reduction of cost, and the easier quality control over the samples.

### SUMMARY OF THE INVENTION

In view of the aforesaid drawbacks of the prior art, it is an objective of the disclosure to provide a polymerase chain reaction (PCR) device comprising at least one holder which at least one reactor tube is separately, detachably disposed at to dispense with the need to arrange multiple reactor tubes on the at least one holder by batch. Moreover, to enable the reactor tubes to share a heat source and thus reduce power consumption, the at least one holder is arranged on a conveying module for driving the reactor tubes on the holders to move synchronously, so as to form a cyclic device.

To achieve the above and other objectives, an embodiment of the disclosure provides a PCR device, comprising:
a conveying module, comprising:
   a bench;
   a rail being loop-style, allowing the bench to move along the rail; and
   at least one holder arranged on the bench, allowing at least one reactor tube to be detachably disposed at the at least one holder; and
a heat supply module, comprising at least one heat supply block for adjusting a temperature of the at least one reactor tube.

Preferably, the holders are in the number of 1 to 100.

Preferably, the heat supply module further comprises a heat-transfer medium and an output portion.

Preferably, the output portion stores and/or outputs the heat-transfer medium, the heat supply blocks adjust a temperature of the heat-transfer medium, and the output portion connects to the heat supply blocks by a pipeline to enable flow of the heat-transfer medium.

Preferably, the heat-transfer medium is a gas or a liquid.

Preferably, some of the heat supply blocks share the same output portion.

Preferably, the heat supply module further comprises a valve for controlling the contact of the heat-transfer medium with the at least one reactor tube.

Preferably, the PCR device further comprises a control module for controlling an operation mode of the conveying module and controlling a heat supply mode of the heat supply module.

Preferably, the operation mode of the conveying module comprises a number of stations that a first holder stops at, and a duration for which the first holder stays at each of the stations before the first holder returns to an initial position upon completion of one cycle of operation of the conveying module.

Preferably, the stations which the at least one holder stops at are in the number of 0 to 100.

Preferably, the duration for which the at least one holder stays at each of the stations is 1 to 300 seconds.

Preferably, the at least one holder stops at the same number of stations for the same duration at each of the stations.

Preferably, one PCR cycle takes place while the at least one holder is staying at each of the stations.

Preferably, the heat supply mode comprises a heat supply temperature and a heat supply duration.

Preferably, the heat supply temperature is sequentially 92 to 96 °C, 45 to 70 °C and 67 to 77 °C in a denaturation phase, an annealing phase and an extension phase during one PCR cycle, respectively.

Preferably, the heat supply duration for a denaturation phase, an annealing phase and an extension phase during one PCR cycle totals 1 to 300 seconds.

Preferably, the heat supply module has at least one heat supply mode.

Preferably, the heat supply module adjusts a temperature of a first portion of holders at a first timepoint with a first heat supply mode and adjusts a temperature of a second portion of holders with a second heat supply mode, the first heat supply mode and the second heat supply mode are different in at least one of a heat supply temperature and a heat supply duration.

Preferably, the PCR device further comprises a driving module for providing a power required to drive the bench.

Preferably, the PCR device further comprises a testing module for testing a product content in the at least one reactor tube.

The aforesaid and other objectives, features and advantage of the disclosure are rendered clear by referring to the detailed description below, preferred embodiments and accompanying drawings.

### BRIEF DESCRIPTION OF THE DRAWINGS

Plenty specific details are provided in the detailed description below to explain the disclosure and enable persons skilled in the art to fully understand the disclosed ways of implementing the disclosure. However, obviously, one or more ways of implementing the disclosure are workable without the aforesaid specific details. In another situation, conventional structures and process flows are schematically depicted by the accompanying drawings for the sake of brevity.

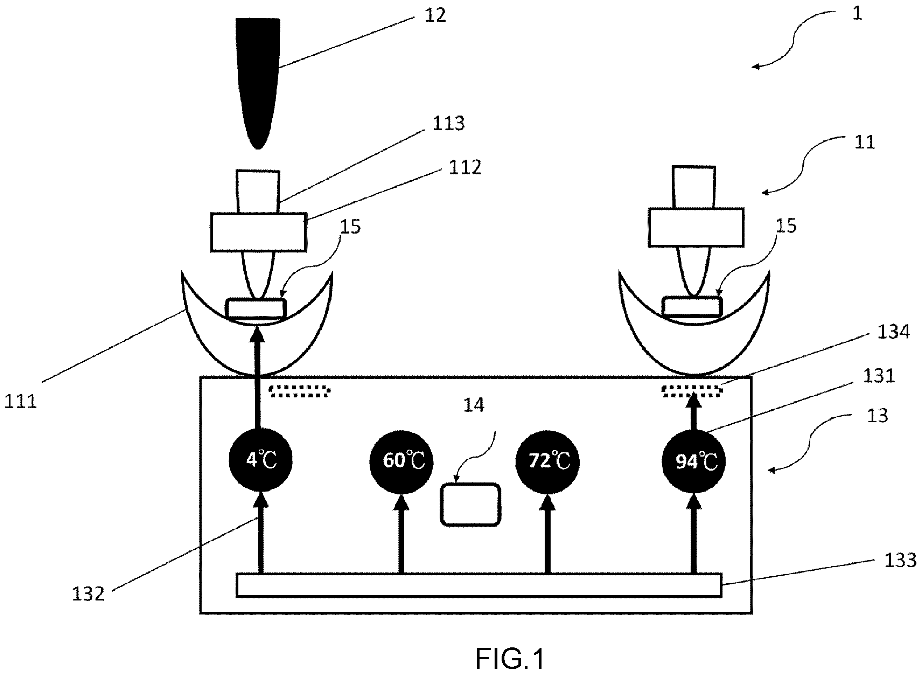

FIG. 1 is a cross-sectional view of a PCR device of the disclosure.

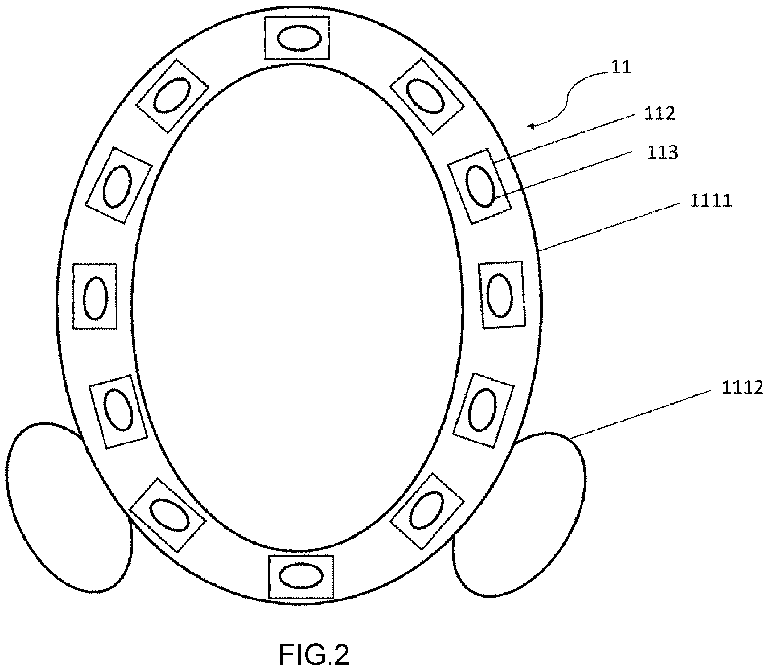

FIG. 2 is a top view of the PCR device of the disclosure.

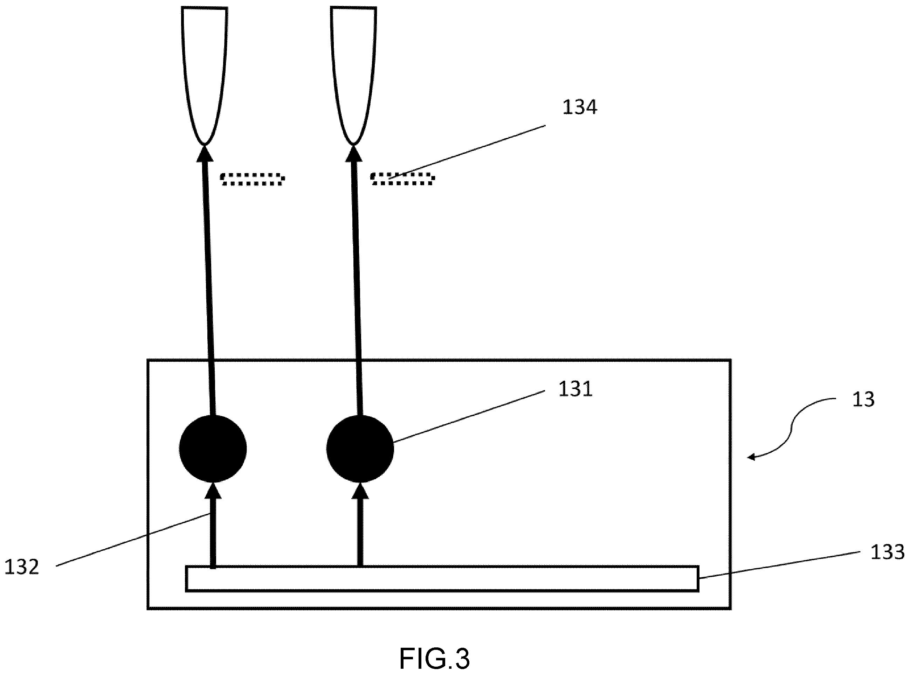

FIG. 3 is a schematic view of a heat supply module of the PCR device of the disclosure. In the heat supply module shown in FIG. 3, one holder is connected to one heat supply block. FIG. 3 shows two heat supply blocks with a temperature that varies to supply heat to two corresponding reactor tubes, respectively. The other heat supply blocks, holders and reactor tubes are not shown in FIG. 3.

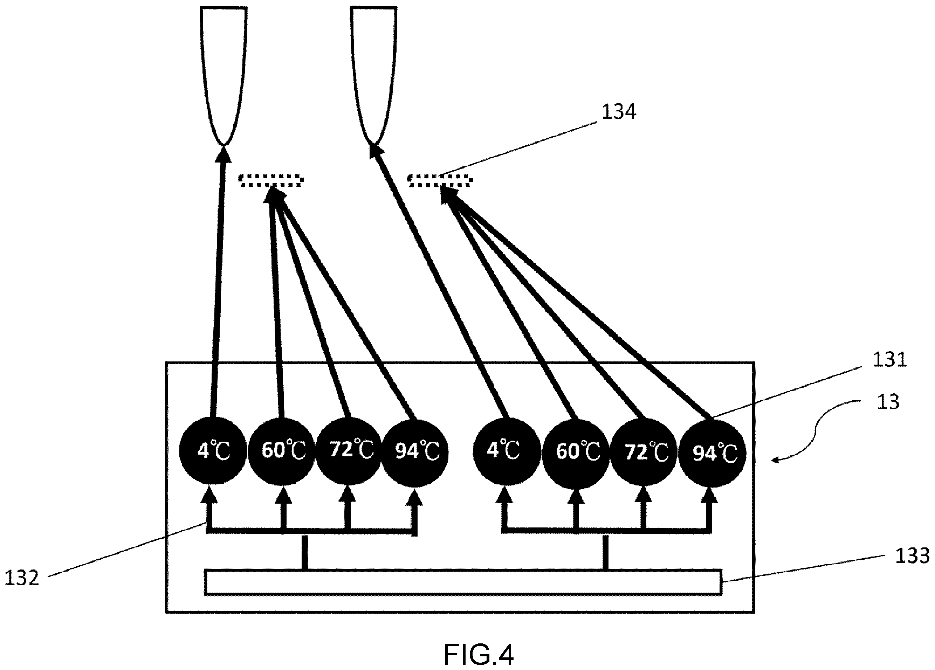

FIG. 4 is a schematic view of the heat supply module of the PCR device of the disclosure. In the heat supply module shown in FIG. 4, one holder is connected to one heat supply block. FIG. 4 shows that a plurality of heat supply blocks within each of the two heat supply block group differ in temperature, but their temperatures do not change, supplying heat of a first temperature (4°C) to corresponding reactor tubes, respectively. The other heat supply blocks, holders and reactor tubes are not shown in FIG. 4.

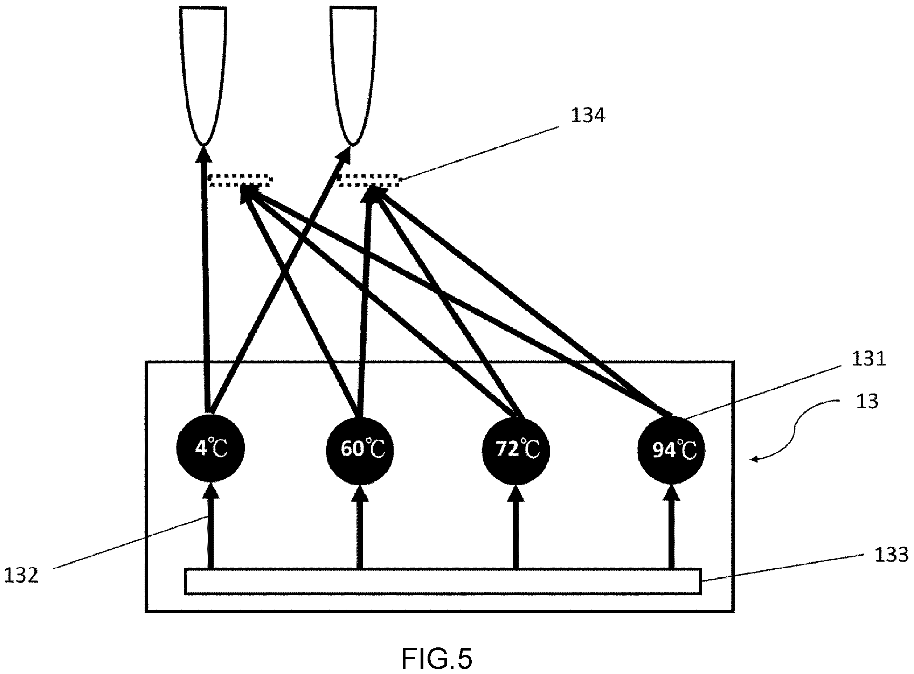

FIG. 5 is a schematic view of the heat supply module of the PCR device of the disclosure. In the heat supply module shown in FIG. 5, a plurality of holders are connected to the same heat supply block group. FIG. 5 shows that a plurality of heat supply blocks within one heat supply block group differ in temperature, but their temperatures do not change, supplying heat of a first temperature (4°C) to two reactor tubes, respectively. The other heat supply blocks, holders and reactor tubes are not shown in FIG. 5.

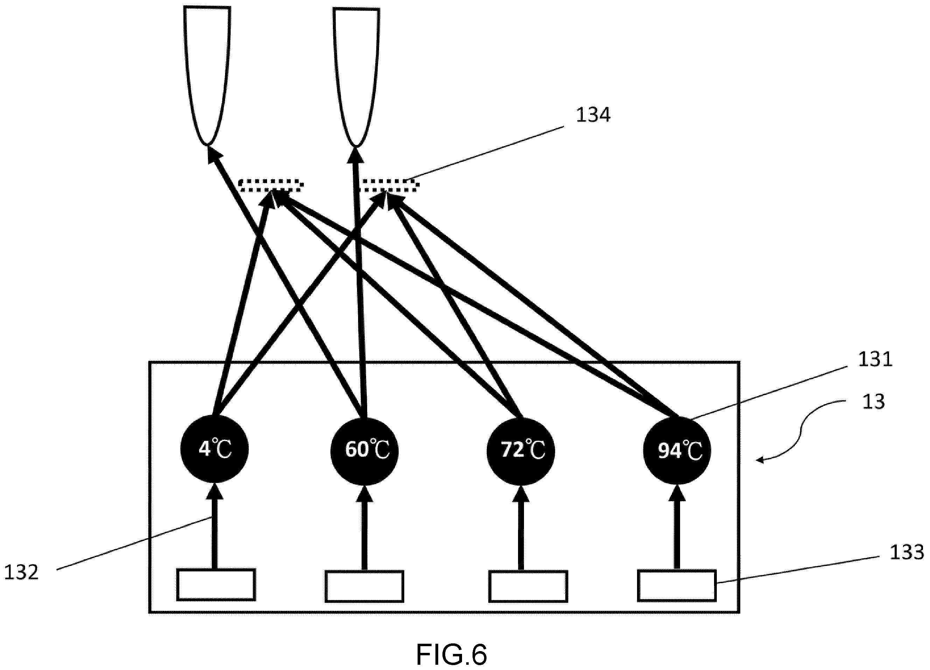

FIG. 6 is a schematic view of the heat supply module of the PCR device of the disclosure. In the heat supply module shown in FIG. 6, a plurality of holders are connected to the same heat supply block group, and a plurality of output portions are connected to different heat supply blocks. FIG. 6 shows that one heat supply block group is identical to its counterpart in FIG. 5, but supplying heat of a second temperature (60°C) to two reactor tubes, respectively. The other heat supply blocks, holders and reactor tubes are not shown in FIG. 6.

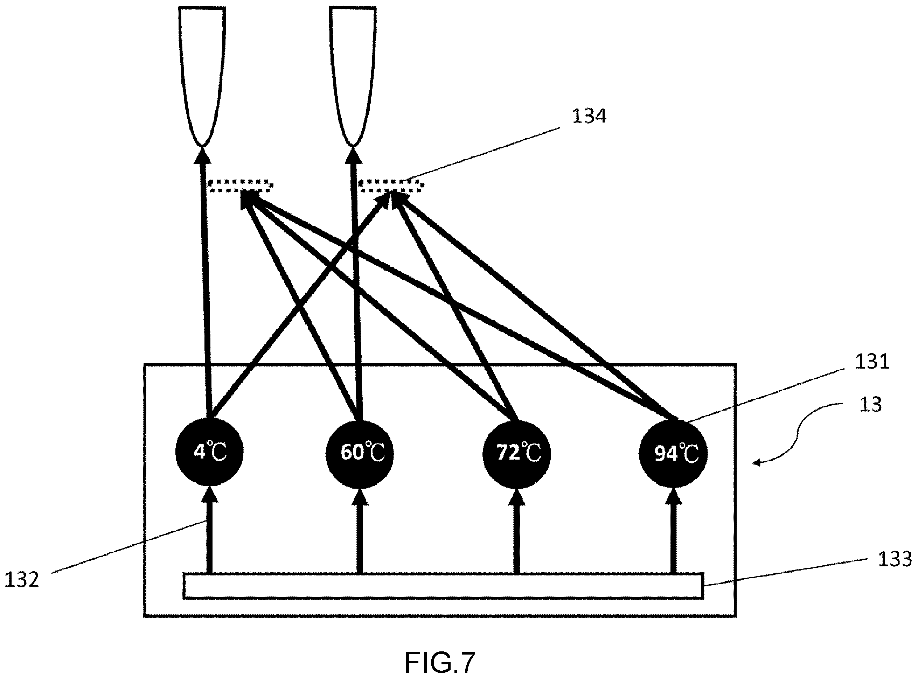

FIG. 7 is a schematic view of the heat supply module of the PCR device of the disclosure. In the heat supply module shown in FIG. 7, a plurality of holders are connected to the same heat supply block group. FIG. 7 shows that one heat supply block group is identical to its counterpart in FIG. 5, but supplying heat of a first temperature (4°C) to a first reactor tube (on the left side in the drawing) and heat of a second temperature (60°C) to a second reactor tube (on the right side in the drawing). The other heat supply blocks, holders and reactor tubes are not shown in FIG. 7.

### DETAILED DESCRIPTION OF THE EMBODIMENTS

Embodiments of the disclosure are depicted by accompanying drawings and described in detail below. The embodiments can be implemented in any form, but it is not the only form in which specific embodiments of the disclosure can be implemented or applied. Thus, the aforesaid form regarding implementing the specific embodiments of the disclosure should not be interpreted in a way to place limitations on the aforesaid embodiments. The ways of implementing the disclosure encompass features of specific embodiments and steps of methods of constructing and operating the specific embodiments and their sequences. However, identical or equivalent functions and step sequences may also be achieved by any other specific embodiments. By contrast, the purpose of the embodiments is to disclose the disclosure thoroughly and completely to sufficiently explain to persons skilled in the art the spirit of the disclosure. Similar reference numerals used in the accompanying drawings denote similar elements. In the description below, conventional functions or structures are not described in detail in order not to repeatedly mention unnecessary details in the embodiments.

Unless otherwise defined, every technical term or jargon used herein carries the same meaning as commonly understood by persons skilled in the art; in the event of a conflict therebetween, this definition-containing specification shall prevail.

Barring a contextual conflict, every singular noun used herein encompasses the plural form of the noun, and every plural noun used herein encompasses the singular form of the noun. The expressions "at least one" and "one or more" used herein and in the appended claims carry the same meaning, i.e., one, two, three or more.

Although approximation is applied to all the numerical ranges and parameters for defining a wider scope of the claimed disclosure, related numerical values used in specific embodiments are herein expressed as precisely as possible. However, every numerical value inherently and inevitably comes with a standard deviation that arises from a related testing method. The word "around" used herein usually describes a specific numerical value or range and indicates that the actual numerical value or range is greater than or less than the specific numerical value or range by 10%, 5%, 1% or 0.5%. Alternatively, the word "around" suggests that the actual numerical value has an acceptable error of the mean, depending on a consideration taken into account by persons skilled in the art. In addition to the embodiments or unless otherwise specified, all ranges, amounts, numerical values and percentages (for example, descriptive of an amount of a required material, a duration, a temperature, an operation requirement, a ratio and the like) used herein are modified by the word "around". Therefore, unless oppositely specified, all numerical values and parameters disclosed herein and in the appended claims are approximate numerical values and are subject to changes as needed. The numerical values and parameters must be, at the very least, interpreted as numerical values characterized by specific significant figures and obtained by a general carry system. In this regard, a numerical range is expressed as starting from an endpoint and ending at another endpoint or as lying between two endpoints. Unless otherwise specified, every numerical range includes endpoints.

An embodiment of the disclosure provides a polymerase chain reaction (PCR) device, comprising a conveying module and a heat supply module. In an embodiment of the disclosure, the conveying module comprises a rail, a bench, and at least one holder.

In an embodiment, the rail is loop-style. The loop-style rail is a rail that means the start and the end of the rail are the same position. In an embodiment, the bench moves along the rail. The at least one holder is arranged on the bench and holds a reactor tube, and thus the reactor tube moves together with the bench along the rail. The loop-style rail of the disclosure enables the reactor tubes to correspond in position to different heat supply blocks respectively while moving along the rail to achieve temperature adjustment. In an embodiment, the rail is divided into a reaction zone and a temporary storage zone by function. A reaction occurs in the reactor tubes while the reactor tubes are passing the reaction zone but not while passing the temporary storage zone. The temporary storage zone is further divided into a first temporary storage zone in which samples can be put to undergo the reaction and a second temporary storage zone from which samples having finished the reaction can be removed.

Conventional batch devices each contain a large number of samples to undergo a reaction in a batch in order to obtain the results about plenty of samples during a short period of time. However, a new batch of samples have to wait their turn until the preceding batch of samples have finished several or a few dozen PCR cycles; as a result, the new batch of samples cannot start to undergo a reaction as soon as possible or immediately upon their arrival. If the samples are STAT samples, they are required by the conventional batch devices to spend much time waiting their turn, thereby delaying the acquisition of results. To deal with STAT samples, the disclosure provides holders that contain a small number of samples each. Thus, according to the disclosure, the STAT samples can be loaded to a bench as soon as possible to undergo a reaction, provided that the number of samples in each batch is reduced or even reduced to one.

In an embodiment, the at least one holder is arranged on the bench. In a preferred embodiment, the holders arranged on the bench are equally spaced apart from each other. In an embodiment, the holders are in the number of 1 to 100. In an embodiment, the conveying module has 60 holders each holding one reactor tube, and thus 60 reactor tubes are disposed in the conveying module. Thus, as many as 60 reactor tubes are undergoing a PCR reaction on the PCR device at the same time. Preferably, the holders are in the number of 1, 5, 10, 15, 20, 30, 40, 50, 60, 70, 80, 90 or 100 approximately, depending on the number of samples to undergo a PCR reaction on the PCR device.

In an embodiment, the at least one reactor tube is detachably disposed at the at least one holder. In a preferred embodiment, the holder has a securing element that can be fastened to fix the reactor tube in place and prevent the detachment of the reactor tube from the holder or the vibration of the reactor tube in the holder. In an embodiment, the disclosure is not restrictive of the direction from which the reactor tube enters the conveying module. In a preferred embodiment, for convenience sake, the reactor tube enters the conveying module from the direction of the upper side of the conveying module, so as to be placed on or fixed to the holder on the conveying module. In an embodiment, the reactor tube is inserted into the holder.

In an embodiment, the heat supply module is disposed on at least one side, for example, the upper side, lower side, left side and/or right side, of the conveying module, but the disclosure is not limited thereto. In a preferred embodiment, for convenience sake, the heat supply module is disposed on the other side of the conveying module and corresponds in position to the reactor tube. In a specific embodiment, when the reactor tube enters the conveying module from the direction of the upper side of the conveying module, the heat supply module is disposed on the lower side of the conveying module. In a feasible embodiment, the heat supply module is centrally disposed at the conveying module to supply heat to the holder completely and efficiently and thereby adjust the temperature of the reactor tube. In an embodiment of the disclosure, the heat supply blocks adjust the temperature of the at least one reactor tube. The heat supply module's supplying heat to the reactor tube entails supplying heat of a temperature higher than the initial temperature of the reactor tube to raise the temperature of the reactor tube and supplying heat of a temperature lower than the initial temperature of the reactor tube to lower the temperature of the reactor tube. Therefore, the heat supply module's supplying heat to the reactor tube is not restricted to heating up the reactor tube to raise its temperature but also includes cooling down the reactor tube to lower its temperature.

In an embodiment of the disclosure, the styles of the operation of the heat supply module include heat supply block style, liquid-cooled style and air-cooled style. The aforesaid three styles of the operation of the heat supply module are explained below.

### Heat supply block style

In an embodiment of the disclosure, the heat supply block style refers to a heating technique employed by the heating blocks in a conventional PCR module. The heat supply block style requires the direct contact between a heat supply block and a holder in order for heat to be supplied to the reactor tube. The heat supply module of heat supply block style comprises at least one heat supply block. In an embodiment, one holder on the rail corresponds in position to one heat supply block, the heat supply blocks change their temperature to cause reactants in the reactor tube to undergo a plurality of steps. In another embodiment, one holder on the rail corresponds in position to a heat supply block group, and the heat supply block group comprises a plurality of heat supply blocks which differ in temperature but never change their temperatures, allowing one heat supply block to cause reactants in the reactor tube to undergo one single step.

Regarding the heat supply module of heat supply block style, the PCR device further comprises a vertically moving module for lifting or lowering the conveying module. When the vertically moving module lifts the conveying module, the holders disposed on the conveying module move away from the heat supply blocks such that the heat supply blocks are not in contact with the holders. By contrast, when the vertically moving module lowers the conveying module, the holders disposed on the conveying module move toward the heat supply blocks to allow the heat supply blocks to come into contact with the holder. Unlike the aforesaid PCR device comprising the vertically moving module for lifting or lowering the conveying module, another PCR device in another aspect comprises a vertically moving module for lifting or lowering the heat supply module. These two PCR devices operate by the same principle, as the contact between each holder and the corresponding heat supply block depends on whether it is the conveying module or the heat supply module whose position is adjusted with the vertically moving module. In a specific aspect, the vertically moving module is a robotic arm for gripping the holder to cause the holder to come into contact with or move away from the heat supply blocks, and the robotic arm comprises a major arm and a minor arm for fine-tuning its movement and position.

### Liquid-cooled style

The liquid-cooled style heat supply module comprises a heat-transfer medium and an output portion. The heat-transfer medium is adapted to come into contact with a holder and is a liquid, such as water, oil, any thermally conductive liquid-state medium or a combination thereof. In an embodiment, the output portion stores and/or outputs the heat-transfer medium. In an embodiment, the output portion is connected to the heat supply blocks by a pipeline, and the heat supply blocks are connected to the holders by a pipeline.

In an embodiment, the liquid-cooled style heat supply module comprises one output portion connected to the heat supply blocks of different temperatures by a pipeline. For instance, when the holders are in the number of 60, the heat-transfer medium outputted from the output portion passes the first pipeline, the second pipeline, the third pipeline, ... the 60th pipeline to come into contact with the first heat supply block, the second heat supply block, the third heat supply block, ... the 60th heat supply block, respectively.

In another embodiment, the liquid-cooled style heat supply module comprises a plurality of output portions connected to the heat supply blocks of the same temperature by a pipeline, respectively. For instance, when the holders are in the number of 60, the heat supply blocks (heat supply block group) are in the number of four and of different temperatures, the heat-transfer medium outputted from four output portions to pass the first pipeline, the second pipeline, the third pipeline, and the fourth pipeline comes into contact with the first heat supply block, the second heat supply block, the third heat supply block, and fourth heat supply block, respectively. In a specific embodiment, the output portions are reservoirs. The plurality of output portions of the liquid-cooled style heat supply module are advantageous in that the heat-transfer medium in the same output portion certainly moves to the heat supply blocks of the same temperature, so that the heat-transfer medium of different output portion will not converge, to render it easy to recycle and output the heat-transfer medium without the hassle of making a big temperature adjustment.

The liquid-cooled style heat supply module comprises at least one heat supply block. In an embodiment, one holder is connected to one heat supply block by a pipeline, and the heat supply block varies its temperature to cause reactants in the reactor tube to undergo a plurality of steps. In another embodiment, one holder is connected to a heat supply block group by a pipeline, and the heat supply block group comprises a plurality of heat supply blocks which differ in temperature but never change their temperatures, allowing one heat supply block to cause reactants in the reactor tube to undergo one single step. In yet another embodiment, a plurality of holders are connected to the same heat supply block or heat supply block group by a pipeline and thus share the heat supply block or heat supply block group.

The liquid-cooled style heat supply module further comprises a valve. The heat-transfer medium, adapted to come into contact with an object of a specific temperature, comes into contact with the at least one reactor tube under the control of the valve. In an embodiment, the valve is of a shape similar to that of a notched knob of a pepper shaker and can be opened and closed in a way like turning the knob. When the notched portion of the knob corresponds in position to the pipeline, the heat-transfer medium is in contact with the holders. By contrast, when the non-notched portion of the knob corresponds in position to the pipeline, the heat-transfer medium is prevented from coming into contact with the holders. In an embodiment, the valve is of a shape similar to that of a spring-equipped marble and is opened and closed by pressing the marble. When a holder corresponds in position to the marble, the holder presses the marble downward, the heat-transfer medium comes into contact with the holder. By contrast, when the holder does not correspond in position to the marble, the marble springs up and thereby blocks the pipeline, preventing the heat-transfer medium from coming into contact with the holder. Depending on its type, the valve is opened and closed adjustably under the control of a control module selectively.

In an embodiment, the liquid-cooled style heat supply module comprises a heating unit. The heating unit is disposed between the valve and the holder and is of a shape similar to that of a sink of a wash stand. Specifically speaking, a drain outlet is disposed at the bottom of the heating unit such that the heat-transfer medium is discharged via the drain outlet and accumulated in the sink, allowing the holder to be immersed in the accumulated heat-transfer medium. In an embodiment, a pump is disposed at one end of the output portion as opposed to the other pipeline-connected end of the output portion to output the heat-transfer medium by pressurization. In another embodiment, a vacuum device is disposed at one end of the heating unit as opposed to the other pipeline-connected end of the heating unit to output the heat-transfer medium under a negative pressure.

In the embodiment of the liquid-cooled style, the heat-transfer medium takes a route as follows: the heat-transfer medium is outputted from an output portion under a pressure exerted by a pump; the heat-transfer medium flows to heat supply blocks via a pipeline; the heat supply blocks adjust the temperature of the heat-transfer medium; the heat-transfer medium flows to a valve via the pipeline; the valve is opened to allow the heat-transfer medium to enter a heating unit to come into contact with a holder; and the heat-transfer medium is recycled and returned to the output portion.

### Air-cooled style

The air-cooled style heat supply module comprises a heat-transfer medium and an output portion. The heat-transfer medium is adapted to come into contact with a holder and is a gas, such as air, a noble gas or a combination thereof. In an embodiment, the output portion stores and/or outputs the heat-transfer medium. In an embodiment, the output portion is connected to the heat supply blocks by a pipeline, and the heat supply blocks are connected to the holders by a pipeline.

In an embodiment, the air-cooled style heat supply module comprises one output portion, and the output portion is connected to the heat supply blocks of different temperatures (with equal total amounts of air current per unit time) by a pipeline. For instance, when the holders are in the number of 60, the heat-transfer medium outputted from the output portion passes the first pipeline, the second pipeline, the third pipeline, ... the 60th pipeline to come into contact with the first heat supply block, the second heat supply block, the third heat supply block, ... the 60th heat supply block, respectively. The air-cooled style heat supply module comprising one single output portion ensures the following: all the heat-transfer mediums moved to the pipelines are equal in quantity and quality and thus conducive to the quality control of the PCR device; only one temperature-controlling valve is ON at a station at the same timepoint; and the same total number of instances of opening and closing the valve and the fixed total amount air current consumption per unit time.

In another embodiment, the air-cooled style heat supply module comprises a plurality of output portions, and the plurality of output portions are connected to the heat supply blocks of the same temperature by a pipeline, respectively. For instance, if the holders are in the number of 60, the heat supply blocks (heat supply block group) are in the number of four, and the heat supply blocks are of different temperatures, then four output portions are provided to output the heat-transfer medium and allow the heat-transfer medium to pass the first pipeline, the second pipeline, the third pipeline, and the fourth pipeline so as to come into contact with the first heat supply block, the second heat supply block, the third heat supply block, and the fourth heat supply block, respectively. In a specific embodiment, the output portion is a fan, and the fan rotates to output a gas functioning as the heat-transfer medium. In an embodiment, the fan is disposed at one end of the heat supply block as opposed to the other holder-connected end of the heat supply block.

The air-cooled style heat supply module comprises at least one heat supply block. In an embodiment, one holder is connected to one heat supply block by a pipeline, and the heat supply block varies its temperature to cause reactants in the reactor tube to undergo a plurality of steps. In another embodiment, one holder is connected to a heat supply block group by a pipeline, and the heat supply block group comprises a plurality of heat supply blocks which differ in temperature but never change their temperatures, allowing one heat supply block to cause reactants in the reactor tube to undergo one single step. In yet another embodiment, a plurality of holders are connected to one heat supply block or heat supply block group by a pipeline and thus share one heat supply block or heat supply block group.

The air-cooled style heat supply module further comprises a valve. The heat-transfer medium, adapted to come into contact with an object of a specific temperature, comes into contact with the at least one reactor tube under the control of the valve. In an embodiment, the valve is of a shape similar to that of a notched knob of a pepper shaker and can be opened and closed in a way like turning the knob. When the notched portion of the knob corresponds in position to the pipeline, the heat-transfer medium is in contact with the holders. By contrast, when the non-notched portion of the knob corresponds in position to the pipeline, the heat-transfer medium is prevented from coming into contact with the holders. In an embodiment, the valve is of a shape similar to that of a spring-equipped marble and is opened and closed by pressing the marble. When a holder corresponds in position to the marble, the holder presses the marble downward, allowing the heat-transfer medium to come into contact with the holder. By contrast, when the holder does not correspond in position to the marble, the marble springs up and thereby blocks the pipeline, preventing the heat-transfer medium from coming into contact with the holder. Depending on its type, the valve is opened and closed adjustably under the control of a control module selectively.

In an embodiment, a pump is disposed at one end of the output portion as opposed to the other pipeline-connected end of the output portion to output the heat-transfer medium by pressurization. In another embodiment, a vacuum device is disposed at one end of the heating unit as opposed to the other pipeline-connected end of the heating unit to output the heat-transfer medium under a negative pressure. In a specific embodiment, the fan of the output portion can be replaced with an air-extracting or vacuum device. The air-extracting or vacuum device is connected to the holders to draw or extract the air from the holder-end, allowing the air at the heat supply block end to pass the heat supply blocks before moving toward the holders.

In the embodiment of the air-cooled style heat-transfer medium takes a route as follows: the heat-transfer medium is outputted from an output portion; the heat-transfer medium flows to heat supply blocks via a pipeline; the heat supply blocks adjust the temperature of the heat-transfer medium; the heat-transfer medium flows to a valve via the pipeline; the valve is opened to allow the heat-transfer medium to come into contact with a holder; and the heat-transfer medium is discharged or recycled. The air-cooled style heat supply module comprising one single output portion ensures the following: all the heat-transfer mediums moved to the pipelines are equal in quantity and quality and thus conducive to the quality control of the PCR device; only one temperature-controlling valve is ON at a station at the same timepoint; and the same total number of instances of opening and closing the valve and the fixed total amount air current consumption per unit time.

In an embodiment of the disclosure, the air-cooled style heat supply module further comprises a control module for controlling an operation mode of the conveying module. The operation mode of the conveying module comprises the number of stations (to be specific, the total number of stations) a first holder stops at and the duration for which the first holder stays at each of the stations before the first holder returns to an initial position upon completion of one cycle of operation of the conveying module. In an embodiment, the stations at which the at least one holder stops are in the number of 0 to 100. In an embodiment, the duration for which the at least one holder stays at each of the stations is 1 to 300 seconds.

On condition that the stations at which the at least one holder stops are in the number of 0, the operation mode of the conveying module is continuous. Thus, the conveying module operates continuously to prevent the holder from stopping at any stations. When the number of the stations at which the at least one holder stops is a positive integer greater than 0, the operation mode of the conveying module is intermittent. Thus, the operation of the conveying module is suspended to cause the holder to stop and stay at the station. The disclosure is not restrictive of a feasible range of the number of stations at which the at least one holder can stop at before the same holder returns to an initial position upon completion of one cycle of operation of the conveying module. The number of stations at which the at least one holder can stop at is 1, 5, 10, 15, 20, 30, 40, 50, 60, ... 100.

In an embodiment of the disclosure, the at least one holder stops at the same number of stations for the same duration at each of the stations. The disclosure adopts a cyclic design to enable all the holders disposed on the conveying module to move together with the conveying module in operation. Therefore, the holders stop at the same number of stations for the same duration at each of the stations spontaneously.

Every one PCR cycle comprises a denaturation phase, an annealing phase and an extension phase, and in these phases a rection occurs at different temperatures, respectively. In an embodiment, the at least one holder staying at one station undergoes one phase of the PCR cycle and thus has the same temperature while staying at the station. For instance, the at least one holder staying at one station undergoes pre-PCR. In another embodiment, the at least one holder staying at one station undergoes a plurality of phases of the PCR cycle and thus changes its temperature to different temperatures while staying at the station.

In yet another embodiment, the at least one holder undergoes one PCR cycle in its entirety while staying at one station and thus changes its temperature to the different temperatures of all the phases of the PCR cycle while staying at the station. The number of stations a holder stops at before the holder returns to an initial position upon completion of one cycle of operation of the conveying module is equal to the number of PCR cycles which the reactor tubes disposed on the holders undergo. With all the holders moving along the rail synchronously, not only do the holders spend the same amount of time staying at each of the stations, but the holders also undergo the reaction at each of the stations for the same duration. Thus, the number of stations a holder stops at before the holder returns to an initial position upon completion of one cycle of operation of the conveying module is greater than the number of PCR cycles which the reactor tubes disposed on the holders undergo.

Regarding the heat supply block style, liquid-cooled style or air-cooled style in an aspect, when one holder corresponds in position to one heat supply block, the implementation of one PCR cycle requires the heat supply block to change its temperature in order to carry out different phases. However, when one holder corresponds in position to one or more heat supply block groups, as disclosed in an embodiment of the disclosure, the heat supply module at least comprises the first heat supply block, the second heat supply block, and the third heat supply block for supplying heat of the first temperature, the second temperature, and the third temperature required for the denaturation phase, annealing phase, and extension phase in one PCR cycle, respectively, with a difference between the first temperature, the second temperature, and the third temperature. Therefore, the holders can switch quickly between different temperatures. Unlike the aspect where only one heat supply block corresponds in position to the holders, this aspect saves the time otherwise spent on changing the temperature of one single heat supply block with a view to carrying out different phases.

In an embodiment of the disclosure, the PCR device further comprises a control module for controlling a heat supply mode of the heat supply module, and the heat supply mode comprises a heat supply duration and a heat supply temperature. The heat supply mode is adjustable according to the characteristics of samples and nucleic acid polymerases. In an embodiment, the heat supply temperatures required for a denaturation phase, an annealing phase and an extension phase in a PCR cycle are 92 to 96 °C, 45 to 70 °C or 67 to 77 °C, respectively. In an embodiment, the heat supply durations in the denaturation phase, the annealing phase and the extension phase are 0.25 to 75 seconds, 0.25 to 150 seconds and 0.5 to 75 seconds, respectively, and the total heat supply duration of one PCR cycle is around 1 to 300 seconds.

In an embodiment, the heat supply module has at least one heat supply mode. The heat supply module adjusts the temperature of the first portion of the holders in a first heat supply mode at the first timepoint and adjusts the temperature of the second portion of the holders in a second heat supply mode. The first heat supply mode and the second heat supply mode are different from each other in at least one of the heat supply temperature and the heat supply duration. Specifically speaking, in the different phases of the PCR cycle, the first holder requires heat supply temperatures of 95°C, 60°C or 72°C, respectively, and heat supply durations of 15 seconds, 30 seconds or 15 seconds, respectively, with the 60th holder requiring the same heat supply temperature as the first holder but the heat supply durations being adjusted and changed to 10 seconds, 20 seconds or 30 seconds, respectively. It is because the sample in the first reactor tube disposed on the first holder stops at the first station to undergo the first PCR cycle reaction, then moves together with the conveying module in operation to the next station to undergo the PCR cycle, and so on until it stops at the 59th station to undergo the 59th PCR cycle; thus, the amount of nucleic acids in the nucleic acid sample in the first reactor tube which is going to stop at the 60th station soon is a multiple of the amount of nucleic acids in the initial sample in the first reactor tube staying at the first station, allowing the heat supply duration of the denaturation phase to be further shortened. The heat supply mode of some of the holders of the heat supply module of the PCR device of the disclosure can be adjusted as needed, for example, providing a third heat supply mode that differs from the first heat supply mode and the second heat supply mode in at least one of the heat supply temperature and the heat supply duration.

In an embodiment, the PCR device further comprises a driving module for providing a power required to drive the bench. In a feasible embodiment, the driving module comprises a motor and a power transmission component. The motor outputs a power, and then the power transmission component provided in the form of a gear or belt transmits the power to the bench to enable the bench to rotate.

In an embodiment, the PCR device further comprises a testing module for testing a product content in the at least one reactor tube. In a feasible embodiment, the testing module is mounted on the conveying module to test the product content in the at least one reactor tube. In a preferred embodiment, a self-contained testing unit is mounted on each reactor tube to monitor the product content in real time.

### Embodiments

FIG. 1 is a cross-sectional view of a PCR device 1 of the disclosure. The PCR device 1 comprises a conveying module 11, a reactor tube 12 and a heat supply module 13. The conveying module 11 comprises a rail 111, a bench 112, and 12 holders 113. The bench 112 receives the power from a driving module 15 in order to operate and move along the rail 111. The holders 113 are arranged on the bench 112. The reactor tube 12 is detachably disposed at the holders 113. The heat supply module 13 is disposed on the lower side of the conveying module 11. The heat supply module 13 comprises 12 heat supply blocks 131, a heat-transfer medium 132, an output portion 133, and a valve 134. The valve 134 controls the contact of heat-transfer medium 132 with the reactor tube 12. The PCR device 1 further comprises a control module 14 for controlling the operation mode of the conveying module 11 and controlling the heat supply mode of the heat supply module 13.

Referring to FIG. 2, there is shown a top view of the PCR device of the disclosure. The PCR device comprises 12 holders 113 arranged on the bench 112.

Referring to FIGS. 3, 4, 5, 6 and 7, there are shown schematic views of heat supply modes in the heat supply module 13 of the PCR device of the disclosure. FIG. 3 shows that the heat supply module 13 comprises two heat supply blocks 131, two heat-transfer mediums 132, and one output portion 133, with each heat supply block 131 being capable of changing to different temperatures. For exemplary sake, the drawing shows two holders each holding a reactor tube (not shown), wherein the holder on the left side is hereunder referred to as the first holder, and the holder on the right side is hereunder referred to as the second holder. The four different temperatures to which the heat supply blocks 131 change are 4°C (for use in amplified nucleic acid product preservation), 60°C (for use in primer annealing), 72°C (for use in DNA polymerization) and 94°C (for use in DNA denaturation), respectively. The heat-transfer mediums 132 are jointly outputted from the output portion 133 and then come into contact with the heat supply blocks 131 of different temperatures via a pipeline, respectively. After having their temperatures adjusted by the heat supply blocks 131, the heat-transfer mediums 132 flow toward the holders via a pipeline. The two valves 134 corresponding in position to the first holder and the second holder open to allow the heat-transfer mediums 132 whose temperatures are adjusted by the heat supply blocks 131.

FIG. 4 shows that the heat supply module 13 comprises two heat supply block groups each having therein four heat supply blocks 131 of different temperatures, four heat-transfer mediums 132, and one output portion 133. For exemplary sake, the drawing shows two holders each holding a reactor tube (not shown), wherein the holder on the left side is hereunder referred to as the first holder, and the holder on the right side is hereunder referred to as the second holder. The four different temperatures to which the heat supply blocks 131 change are 4°C (for use in amplified nucleic acid product preservation), 60°C (for use in primer annealing), 72°C (for use in DNA polymerization) and 94°C (for use in DNA denaturation), respectively. The heat-transfer mediums 132 are jointly outputted from the output portion 133 and then come into contact with the heat supply blocks 131 of different temperatures via pipelines, respectively. After having their temperatures adjusted by the heat supply blocks 131, the heat-transfer mediums 132 flow toward the holders via a pipeline. The two valves 134 corresponding in position to the first holder and the second holder open to allow the heat-transfer mediums 132 whose temperatures are adjusted by the heat supply blocks 131 of a temperature of 4°C but close to shut out the heat-transfer mediums 132 whose temperatures are adjusted by the heat supply blocks 131 of any other temperatures.

FIG. 5 shows that the heat supply module 13 comprises four heat supply blocks 131 of different temperatures, four heat-transfer mediums 132, and one output portion 133. For exemplary sake, the drawing shows two holders each holding a reactor tube (not shown), wherein the holder on the left side is hereunder referred to as the first holder, and the holder on the right side is hereunder referred to as the second holder. The four different temperatures to which the heat supply blocks 131 change are 4°C, 60°C, 72°C and 94°C. The heat-transfer mediums 132 are jointly outputted from the output portion 133 and then come into contact with the heat supply blocks 131 of different temperatures via a pipeline, respectively. After having their temperatures adjusted by the heat supply blocks 131, the heat-transfer mediums 132 flow toward the holders via a pipeline. The two valves 134 corresponding in position to the first holder and the second holder open to allow the heat-transfer mediums 132 whose temperatures are adjusted by the heat supply blocks 131 of a temperature of 4°C but close to shut out the heat-transfer mediums 132 whose temperatures are adjusted by the heat supply blocks 131 of any other temperatures.

Compared with FIG. 5, FIG. 6 shows distinguishing technical features as follows: the heat supply module comprises four output portions 133 connected to the heat supply blocks 131 of different temperatures, respectively; and the two valves 134 corresponding in position to the first holder and the second holder open to allow the heat-transfer mediums 132 whose temperatures are adjusted by the heat supply blocks 131 of a temperature of 60°C but close to shut out the heat-transfer mediums 132 whose temperatures are adjusted by the heat supply blocks 131 of any other temperatures.

Compared with FIG. 5, FIG. 7 shows distinguishing technical features as follows: the valve 134 corresponding in position to the first holder opens to allow the heat-transfer mediums 132 whose temperatures are adjusted by the heat supply blocks 131 of a temperature of 4°C but closes to shut out the heat-transfer mediums 132 whose temperatures are adjusted by the heat supply blocks 131 of any other temperatures; and a valve corresponding in position to the second holder opens to allow the heat-transfer mediums 132 whose temperatures are adjusted by the heat supply blocks 131 of a temperature of 60°C but closes to shut out the heat-transfer mediums 132 whose temperatures are adjusted by the heat supply blocks 131 of any other temperatures.

### First Embodiment

The conveying module of the PCR device comprises 24 holders, allowing 24 samples to be positioned in the holders sequentially in response to the operation of the conveying module so as to undergo the reaction. The operation mode is intermittent, allowing the holders to stop at 24 stations and spend one minute and 45 seconds staying at each of the stations. The heat supply module is air-cooled style and comprises a fan functioning as an output portion and three heat supply blocks. The gas outputted from the fan functions as the heat-transfer medium. The output portion, the heat supply blocks and holders are connected by pipelines to enable the flow of the heat-transfer medium. A valve is disposed on a pipeline between each heat supply block and the corresponding holder to control the contact of the gas with the holder. The gas outputted from the fan flows into three different channels to reach the three heat supply blocks, respectively. Then, the gas to which heat is supplied by each heat supply block is divided to flow into 24 different channels to reach 24 holders, respectively. In the heat supply mode, the three heat supply blocks require heat supply temperatures of 94°C, 60°C, 72°C, respectively, and heat supply durations of 20 seconds, 60 seconds, 25 seconds, respectively. The heat supply mode to each holder is the same. The heat supply duration in the heat supply mode is one minute and 45 seconds in total, synchronizing with the stay duration in the operation mode.

### Second Embodiment

The conveying module of the PCR device comprises 48 holders, allowing 48 samples to be positioned in the holders sequentially in response to the operation of the conveying module so as to undergo the reaction. The operation mode of the conveying module is continuous. Thus, the conveying module operates continuously to prevent the holder from stopping at any stations. Each holder connected to the heat supply module pipeline for one minute. The heat supply module is air-cooled style and comprises a fan functioning as an output portion and three heat supply blocks. The gas outputted from the fan functions as the heat-transfer medium. The output portion, heat supply blocks and holders are connected by a pipeline to enable the flow of the heat-transfer medium. A valve is disposed on a pipeline between each heat supply block and the corresponding holder to control the contact of the gas the holder. The gas outputted from the fan flows into three different channels to reach the three heat supply blocks, respectively. Then, the gas to which heat is supplied by each heat supply block is divided to flow into 48 different channels to reach 48 holders, respectively. In the heat supply mode, the three heat supply blocks require heat supply temperatures of 95°C, 65°C, 77°C, respectively, and heat supply durations of 25 seconds, 55 seconds, 20 seconds, respectively, The heat supply mode to each holder is the same. The heat supply duration in the heat supply mode is one minute 40 seconds in total, synchronizing with the duration of connection to each holder in the operation mode.

### Third Embodiment

The conveying module of the PCR device comprises 60 holders, allowing 60 samples to be positioned in the holders sequentially in response to the operation of the conveying module so as to undergo the reaction. The operation mode is intermittent, allowing the holders to stop at 60 stations and spend one minute staying at each of the stations. The heat supply module is liquid-cooled style and comprises three reservoirs functioning as output portions and three heat supply blocks. The liquid outputted from the reservoirs functions as the heat-transfer medium. The output portions, the heat supply blocks and the holders are connected by a pipeline to enable the flow of the heat-transfer medium. A valve is disposed on a pipeline between each heat supply block and the corresponding holder to control the contact of the liquid with the holder. The liquid outputted from the reservoirs flows into three different channels, respectively, to reach three heat supply blocks, respectively. Then, the liquid to which heat is supplied by each heat supply block is divided to flow into 60 different channels to reach 60 holders, respectively. In the heat supply mode, the three heat supply blocks require heat supply temperatures of 92°C, 55°C, 67°C, respectively, and heat supply durations of 15 seconds, 30 seconds, 15 seconds, respectively. The heat supply module for each holder is the same. The heat supply duration in the heat supply mode is one minute in total, synchronizing with the stay duration in the operation mode.

### Fourth Embodiment

The conveying module of the PCR device comprises 60 holders, allowing 60 samples to be positioned in the holders sequentially in response to the operation of the conveying module so as to undergo the reaction. The operation mode is intermittent, allowing the holders to stop at 60 stations and spend one minute staying at each of the stations. The heat supply module is liquid-cooled style and comprises three reservoirs functioning as output portions and three heat supply blocks. The liquid outputted from the reservoirs functions as the heat-transfer medium. The output portion, heat supply blocks and holders are connected by a pipeline to enable the flow of the heat-transfer medium. A valve is disposed on a pipeline between each heat supply block and the corresponding holder to control the contact of the liquid with the holder. The liquid outputted from the reservoirs flows into four different channels to reach four heat supply blocks, respectively. Then, the liquid to which heat is supplied by each heat supply block is divided to flow into 60 different channels to reach 60 holders, respectively. In the heat supply mode, the four heat supply blocks require heat supply temperatures of 95°C, 55°C, 72°C and 4°C, respectively, and the first to tenth holders require a heat supply temperature of 95°C and a heat supply duration of one minute, so as to perform the step of hot-starting a polymerase chain reaction (PCR) (hot-start PCR) to activate a polymerase in the presence of high heat. The 11th to 55th holders require heat supply temperatures of 95°C, 55°C, 72°C, respectively, and heat supply durations of 15 seconds, 30 seconds, 15 seconds, respectively, with each holder undergoing one PCR cycle, allowing a total of 45 cycles to take place. The 55th to 60th holders require a heat supply temperature of 4°C and a heat supply duration of one minute. The 60 holders in this aspect have a total of three different heat supply modes. The heat supply duration in the heat supply mode is one minute in total, synchronizing with the duration of suspension of operation in the operation mode.

Therefore, the PCR device of the disclosure achieves the objective of loading a small number of samples to the PCR device as soon as possible to undergo a reaction. Unlike conventional batch devices, a cyclic PCR device provided by the disclosure allows holders to move between different stations as a result of the operation of the conveying module and allows samples in reactor tubes disposed on the holders to undergo PCR while the holders are moving between different stations. Arriving samples just need to wait the duration of one PCR cycle in order to be placed on the holders idling in the PCR device to start to undergo the reaction.

The disclosure provides a conveying module for sharing heat supply blocks, heat-transfer mediums and/or output portions to save energy, reduce the required device volume, and save space.

The operation of the conveying module enables the holders to move synchronously. Thus, the reactor tubes spend the same amount of time carrying out one PCR cycle to not only facilitate quality control but also enhance the ease of recording the number of finished cycles. Although the total time taken by the reactor tubes to carry out one PCR cycle is the same, the mode of each step in the PCR cycle can be freely adjusted and controlled, thereby meeting various requirements of different samples.

The above description is illustrative rather than restrictive. All equivalent amendments or changes made to the embodiments of the disclosure without departing from the spirit and scope of the disclosure must be deemed falling within the scope of the appended claims.
- 1: PCR device
- 11: conveying module
- 111: rail
- 1111: reaction zone of the rail
- 1112: temporary storage zone of the rail
- 112: bench
- 113: holders
- 13: heat supply module
- 131: heat supply blocks
- 132: heat-transfer medium
- 133: output portion
- 134: valve
- 14: control modul
- 15: driving module

## Claims

1. A PCR device, comprising:
a conveying module, comprising
a bench;
a rail being loop-style, allowing the bench to move along the rail; and
at least one holder arranged on the bench, allowing at least one reactor tube to be detachably disposed at the at least one holder; and
a heat supply module comprising at least one heat supply block for adjusting a temperature of the at least one reactor tube.

2. The PCR device of claim 1, wherein the holders are in the number of 1 to 100.

3. The PCR device of claim 1, wherein the heat supply module further comprises a heat-transfer medium and an output portion.

4. The PCR device of claim 3, wherein the output portion stores and/or outputs the heat-transfer medium, the heat supply blocks adjust a temperature of the heat-transfer medium, and the output portion connects to the heat supply blocks by a pipeline to enable flow of the heat-transfer medium.

5. The PCR device of claim 3, wherein the heat-transfer medium is a gas or a liquid.

6. The PCR device of claim 3, some of the heat supply blocks share the same output portion.

7. The PCR device of claim 3, wherein the heat supply module further comprises a valve for controlling the contact of the heat-transfer medium with the at least one reactor tube.

8. The PCR device of claim 1, further comprising a control module for controlling an operation mode of the conveying module and controlling a heat supply mode of the heat supply module.

9. The PCR device of claim 8, wherein the operation mode of the conveying module comprises a number of stations a first holder stops at and a duration for which the first holder stays at each of the stations before the first holder returns to an initial position upon completion of one cycle of operation of the conveying module.

10. The PCR device of claim 9, wherein the stations which the at least one holder stops at are in the number of 0 to 100.

11. The PCR device of claim 9, wherein the duration for which the at least one holder stays at each of the stations is 1 to 300 seconds.

12. The PCR device of claim 9, wherein the at least one holder stops at the same number of stations for the same duration at each of the stations.

13. The PCR device of claim 9, one PCR cycle takes place while the at least one holder is staying at each of the stations.

14. The PCR device of claim 8, wherein the heat supply mode comprises a heat supply temperature and a heat supply duration.

15. The PCR device of claim 14, wherein the heat supply temperature is sequentially 92 to 96 °C, 45 to 70 °C and 67 to 77 °C in a denaturation phase, an annealing phase and an extension phase during one PCR cycle, respectively.

16. The PCR device of claim 14, wherein the heat supply duration for a denaturation phase, an annealing phase and an extension phase during one PCR cycle totals 1 to 300 seconds.

17. The PCR device of claim 8, wherein the heat supply module has at least one heat supply mode.

18. The PCR device of claim 17, wherein the heat supply module adjusts a temperature of a first portion of holders at a first timepoint with a first heat supply mode and adjusts a temperature of a second portion of holders with a second heat supply mode, the first heat supply mode and the second heat supply mode are different in at least one of a heat supply temperature and a heat supply duration.

19. The PCR device of claim 1, further comprising a driving module for providing a power required to drive the bench.

20. The PCR device of claim 1, further comprising a testing module for testing a product content in the at least one reactor tube.
